# EUROPÄISCHE PATENTSCHRIFT

(11) **EP 4 534 520 B1**
(45) Veröffentlichungstag und Bekanntmachung des Hinweises auf die Patenterteilung: **03.12.2025**
(21) Anmeldenummer: 23201143.7
(22) Anmeldetag: 02.10.2023
(51) Int. Cl.: C07C 29/151, C07C 31/04, C07C 31/08, C10J 3/48, B01J 19/08

(54) **VERFAHREN UND ANLAGE ZUR HERSTELLUNG ALKOHOLISCHER E-KRAFTSTOFFE AUS HETEROGENEN BIO-KUNSTSTOFF-HALOGENABFALLMISCHUNGEN**
METHOD AND PLANT FOR PRODUCING ALCOHOLIC E-FUELS FROM HETEROGENEOUS BIOPLASTIC HALOGEN WASTE MIXTURES
PROCÉDÉ ET INSTALLATION DE PRODUCTION D'E-CARBURANTS ALCOOLIQUES À PARTIR DE MÉLANGES HÉTÉROGÈNES BIO-PLASTIQUES-HALOGÈNE

(43) Veröffentlichungstag der Anmeldung: 09.04.2025
(73) Patentinhaber: 1to7plastastic GmbH, 14943 Luckenwalde (DE)
(72) Erfinder: KLIEMKE, Hardy, 14943 Luckenwalde (DE)
(74) Vertreter: Vossius & Partner Patentanwälte Rechtsanwälte mbB

(56) Entgegenhaltungen:
- WO-A1-2023/015343
- US-A1- 2014 179 959
- ZHOU RENWU ET AL: "Underwater microplasma bubbles for efficient and simultaneous degradation of mixed dye pollutants", SCIENCE OF THE TOTAL ENVIRONMENT, ELSEVIER, AMSTERDAM, NL, vol. 750, 12 September 2020 (2020-09-12), XP086319548, ISSN: 0048-9697, [retrieved on 20200912], DOI: 10.1016/J.SCITOTENV.2020.142295

## Beschreibung

Die vorliegende Erfindung bezieht sich auf eine Anlage und ein Verfahren zur Herstellung von Alkohol aus heterogenen Bio-Kunststoff-Halogenabfallmischungen. In einer Zeit, in der Abfallmanagement und nachhaltige Energieerzeugung von zentraler Bedeutung sind, bietet diese Erfindung eine innovative Lösung zur Umwandlung von Abfällen in wertvolle Ressourcen.

Häusliche Abfälle bestehen typischerweise aus einer Mischung von biogenen Abfällen und Verpackungsmaterialien. Insbesondere die Vielfalt und Heterogenität der Kunststoffe im Hausmüll erschweren das Recycling. Eine effiziente Trennung dieser Kunststoffe ist oft komplex und nicht immer vollständig möglich, weshalb viele dieser Abfälle in Müllverbrennungsanlagen thermisch verwertet werden.

Es gibt bereits etablierte Verfahren zur Erzeugung von Alkohol-Synthesegasen, als Zwischenschritt zur Erzeugung von Alkoholen, insbesondere aus Biomasse oder Kunststoffen. Beispielsweise beschreibt die Veröffentlichung DE 10 2006 012 313 A1 ein Verfahren zur Reduktion von Kohlenstoffdioxid und Wasser unter Verwendung eines Sauerstoffionenleiters. DE 10 2012 010 542 A1 stellt ein System zur Erzeugung von Synthesegas vor. Die US 2014/179959 A1 beschreibt einen Reformer zum Umwandeln eines Ausgangsbrennstoffs, bspw. Methan, in einen Ausgabebrennstoff, bspw. Methanol. Die dort beschriebenen Lösungen haben jedoch beide ihre eigenen Einschränkungen und Nachteile.

Zusätzlich zu diesen Ansätzen gibt es Prozesse wie die "Pyrolyse".

Es besteht daher nach wie vor die Aufgabe, eine verbesserte Anlage bzw. ein verbessertes Verfahren zur Erzeugung von Alkohol-Synthesegasen, insbesondere aus Biomasse oder Kunststoffen, bzw. eine effiziente, betriebssichere und kostengünstige Lösung zur Herstellung alkoholischer E-Kraftstoffe bereitzustellen. Diese Aufgabe wird durch die Anlage gemäß Anspruch 1 und das Verfahren gemäß Anspruch 6 gelöst.

Die vorliegende Erfindung beruht unter anderem auf der Anwendung des Blasensäulen-Prinzips. Dieses Prinzip nutzt das Verhalten von in einer Flüssigkeit aufsteigenden Blasen, die gereinigt werden, wodurch Pyrolyse-Zwischenprodukte aus dem Gas abgetrennt und erneut dem Plasmalyseprozess zugeführt werden. Dies steigert die Effizienz und Reinheit des erzeugten Alkohol-Synthesegases und erhöht den Umsatz von Abfall zum anvisierten Produkt, dem Alkohol.

Dabei kombiniert die vorliegende Erfindung mehrere aus dem Stand der Technik bekannte Technologien. Sie integriert die Prinzipien der "Plasmalyse", "Blasensäulen", "Alkoholsynthese aus Synthesegas", "Elektrolyse" und "Pyrolyse" in einer neuartigen Weise. Das Ergebnis ist eine kompakte und sichere Anlage sowie ein dazugehöriges Verfahren, das speziell darauf ausgelegt ist, komplexe, heterogene Mischungen häuslicher Abfälle, insbesondere "Bio-Kunststoff-Halogenabfallmischungen", effizient in einen E-Kraftstoff, harmlose Asche oder Schlacke und sauberes Wasser zu zerlegen.

Ein Kerngedanke der Erfindung liegt dabei in ihrer Dualität: Sie dient sowohl als Energiespeicher als auch als Recyclinganlage. Während Abfallenergie als eine der Hauptressourcen genutzt wird, fließt auch elektrische Energie in den Prozess ein, um den Treibstoff zu erzeugen. Dies stellt nicht nur eine innovative Methode zur Abfallverwertung dar, sondern auch eine effiziente Möglichkeit, erneuerbare Energien zu speichern und zu nutzen.

Ein weiterer zentraler Schritt dieses Prozesses ist die Zerkleinerung der Abfallmischung in eine granulare Form, um eine optimale Durchmischung und effektive Reaktionen in den nachfolgenden Verarbeitungsschritten zu gewährleisten. Dieses zerkleinerte Granulat wird dann mit einem Wasserstrom gemischt, um eine Suspension zu bilden, die in den Plasma-Blasensäulenreaktor eingebracht wird.

Innerhalb des Reaktors wird die Suspension durch Plasmaenergie präzise umgewandelt. Das Plasma wird durch die Zugabe von elektrischem Strom erzeugt, wodurch die Abfallpartikel getrocknet, verdampft und in den Plasmazustand überführt werden. Nach der Energieübertragung kondensieren die Plasmablasen und die zuvor dissoziierten Atome rekombinieren sich zu kleineren, leichtflüchtigen Molekülen wie CO, CO2, H2, H2O und CH4, die das Alkoholsynthesegas bilden.

Ein weiterer wichtiger Aspekt der Erfindung ist die präzise Einstellung des C:H- und C:H:O-Verhältnisses, welches auf die Zusammensetzung des Synthesegases abzielt, das den Plasma-Blasensäulenreaktor und den nachfolgenden Halogenwasserstoff-Neutralisator verlässt. Dies wird durch die gezielte Zugabe von Wasser und die Kontrolle des Sauerstoffgehalts mittels eines Sauerstoffabscheiders erreicht. Das C:H:O-Verhältnis ergibt sich aus der chemischen Summenformel des zu erzeugenden Alkohols und bezieht sich auf die Summe aller C-, aller H- und aller O-Atome in der Summenformel. Beispielsweise beträgt das C:H:O-Verhältnis für Methanol (CH3OH beziehungsweise CH4O) 1:4:1 und für Ethanol (C2H5OH beziehungsweise C2H6O) 2:6:1. Es ist zu beachten, dass das C:H:O-Verhältnis der Bio-Kunststoff-Halogenabfallmischung natürlichen Schwankungen unterliegt. Eine Toleranz von jeweils ca. 5 Prozent bezogen auf die Anzahl der H-Atome und der O-Atome ist als erfindungsgemäß zu betrachten. Zudem ist zu betonen, dass Wasser bzw. Wasserdampf bei der Bestimmung des C:H:O-Verhältnisses nicht berücksichtigt wird, da Wasser und Wasserdampf primär als Transportmittel fungieren und somit nicht in die Berechnung des Verhältnisses einfließen.

Das so erzeugte Alkohol-Synthesegas, oft auch einfach als Synthesegas bezeichnet, kann Bestandteile wie Wasserstoff (H2), Kohlenstoffmonoxid (CO), Sauerstoff (O2), Kohlenstoffdioxid (CO2), Wasser (H2O) und Methan (CH4), aber auch Verunreinigungen von Halogenwasserstoffen enthalten. Es dient insbesondere zur Erzeugung eines Alkohols, vorzugsweise Methanol. Nach der Plasmabehandlung strömt das Alkoholsynthesegas in den Halogenwasserstoff-Neutralisator. Halogenwasserstoffe, die gefährliche Verbindungen darstellen, werden in diesem Neutralisator in harmlose Stoffe wie z.B. Salz, Wasser und CO2 umgewandelt und somit effektiv unterdrückt.

Danach strömt das Alkoholsynthesegas weiter in einen Katalysator und wird beim Durchströmen des Katalysators in ein Gemisch aus Alkohol und Wasser transformiert. Bei der anschließenden Trennung dieses Gemisches entstehen verschiedene Stoffströme: Wasser, Alkohol, unvollständig umgesetztes Alkoholsynthesegas, ein sauerstoffreicher Stoffstrom und ein Ausgang für nicht reagierende Gase wie Stickstoff. Dieser sauerstoffreiche Stoffstrom, der aus Wasser, CO2 oder einer Kombination beider bestehen kann, wird dem Sauerstoffabscheider zugeführt, um den Sauerstoffgehalt im System präzise zu regulieren.

Ein besonderer Fokus liegt auf der Rolle des Wassers im Prozess. Wasser dient als Transportmittel und Energiemoderator. Es transportiert die granulären Abfallpartikel als Suspension in und aus dem Plasma-Blasensäulenreaktor. Durch die Verdampfung eines Teils des verwendeten Wassers im Reaktor wird eine hohe Anlagensicherheit gewährleistet, da die hohe Energie des Plasmas nicht bis zur inneren Oberfläche des Reaktors gelangt. Die anschließende Kondensation des Wassers in Katalysator und Trennungssystem führt zu hochreinem Wasser, das für den Sauerstoffabscheider von entscheidender Bedeutung ist. Dieses hochreine Wasser, das hauptsächlich Spuren des Alkohols enthält, wird entweder dem Sauerstoffseparationssystem zugeführt oder aus dem System entfernt. Extern zugeführtes Wasser für die Suspensionsbildung kann auch verschmutztes Abwasser sein.

Die Erfindung integriert auch Stoffrückführungen, wie z.B. die Rückführung des unvollständig umgesetzten Alkoholsynthesegases und Wärmeübertrager, um die Effizienz des Prozesses zu maximieren. Dies gewährleistet nicht nur die Qualität des erzeugten Alkohols, sondern auch die Sicherheit, Stabilität, Effizienz und Umweltfreundlichkeit des gesamten Prozesses.

Zusammenfassend bietet die erfindungsgemäße Vorrichtung eine nachhaltige und effiziente Methode zur Umwandlung von Bio-Kunststoff-Halogenabfallmischungen in E-Kraftstoff, wodurch sie einen bedeutenden Fortschritt im Bereich der Abfallverwertung und nachhaltigen Energieerzeugung darstellt.

Im Kontext der vorliegenden Erfindung bezieht sich der Begriff 'Bio-Kunststoff-Halogenabfallmischung' auf eine Mischung, die sowohl biologische Abfälle als auch Kunststoffabfälle und halogenhaltige Verbindungen enthält. Während die biologischen Abfälle aus natürlichen, organischen Quellen stammen, repräsentieren die Kunststoffabfälle menschengemachte Polymere. Die halogenhaltigen Verbindungen sind oft Verunreinigungen, die durch bestimmte Kunststoffe, wie PVC, oder durch Zusätze wie fluorhaltige Weichmacher in die Mischung gelangen.

Im Kontext der vorliegenden Erfindung bezieht sich der Begriff 'Synthesegas' auf ein vielseitiges Gasgemisch, das primär aus Wasserstoff (H₂), Kohlenstoffmonoxid (CO), Wasser (H₂O), Kohlenstoffdioxid (CO₂) und Methan (CH₄) bestehen kann.

Alkoholsynthesegas stellt eine spezifische Variante des Synthesegases dar, die besonders für die Produktion von Alkoholen, insbesondere Methanol und Ethanol, optimiert ist. Es zeichnet sich durch ein charakteristisches C:H:O-Verhältnis aus, welches sich aus der chemischen Summenformel des gewünschten Alkohols ableitet. Zum Beispiel entspricht das C:H:O-Verhältnis von Methanol (CH₃OH) 1:4:1. Neben den Hauptkomponenten können im Alkoholsynthesegas auch geringe Mengen anderer Gase enthalten sein.

Halogenwasserstoffe sind eine Gruppe von chemischen Verbindungen, die aus Wasserstoff (H) und einem Halogenatom (Fluor, Chlor, Brom, lod oder Astat) bestehen. Die bekanntesten Vertreter dieser Gruppe sind Fluorwasserstoff (HF), Chlorwasserstoff (HCl), Bromwasserstoff (HBr) und lodwasserstoff (HI).

Diese Verbindungen können entstehen, wenn im Abfall enthaltene Halogene, wie beispielsweise Chlor aus PVC oder Fluor aus bestimmten Weichmachern, unter bestimmten Bedingungen mit Wasserstoff reagieren.

Halogenwasserstoffe sind aus mehreren Gründen problematisch:
1. Korrosivität: Halogenwasserstoffe, insbesondere HF und HCl, sind stark korrosiv und können Metalle und andere Materialien angreifen, aus denen Anlagen und Ausrüstungen bestehen. Dies kann zu vorzeitigem Verschleiß und potenziellen Leckagen führen.
2. Toxizität: Einige Halogenwasserstoffe, insbesondere HF, sind hochtoxisch und können bei Kontakt mit der Haut, den Augen oder beim Einatmen schwere Verletzungen verursachen.
3. Umweltauswirkungen: Wenn Halogenwasserstoffe in die Umwelt freigesetzt werden, können sie zu saurem Regen beitragen und Wasserquellen kontaminieren. Dies kann negative Auswirkungen auf Pflanzen, Tiere und den Menschen haben.
4. Störung von Prozessen: In industriellen Prozessen können Halogenwasserstoffe unerwünschte Nebenreaktionen verursachen und die Qualität des Endprodukts beeinträchtigen.

Aufgrund dieser Gefahren ist es wichtig, die Bildung von Halogenwasserstoffen in Prozessen, die Abfälle behandeln, zu minimieren oder effektiv zu kontrollieren und zu neutralisieren.

Im Kontext der vorliegenden Erfindung bezieht sich der Begriff 'Halogenwasserstoff-Neutralisator' auf ein System, das entwickelt wurde, um die Bildung von Halogenwasserstoffen zu verhindern oder zu reduzieren. Diese Halogenwasserstoffe können entstehen, wenn im Abfall enthaltene Halogene, wie Chlor oder Fluor, unter bestimmten Bedingungen reagieren.

Halogenwasserstoff-Neutralisator: Der Halogenwasserstoff-Neutralisator dient primär dazu, gebildete Halogenwasserstoffe, die im Alkoholsynthesegas enthalten sind, unschädlich zu machen. Seine Hauptfunktionen sind:
1. Neutralisation: Der Inhibitor neutralisiert Halogenwasserstoffe, indem er sie in weniger schädliche Verbindungen wie Salze, CO2 oder Wasser umwandelt.
2. Schutz von Anlagen und Ausrüstungen: Er schützt Metalle und andere Materialien vor den korrosiven Wirkungen von Halogenwasserstoffen.
3. Sicherheit und Umweltschutz: Er verhindert die Bildung oder Freisetzung von toxischen Halogenwasserstoffen und trägt so zum Schutz von Arbeitern und der Umwelt bei.

In der hier beschriebenen Anwendung besteht der Halogenwasserstoff-Neutralisator aus Materialien wie Calciumcarbonat oder Natriumcarbonat, die aufgrund ihrer chemischen Eigenschaften besonders wirksam bei der Neutralisation von Halogenwasserstoffen sind.

Ein Plasma-Blasensäulenreaktor erweitert das Konzept der im Stand der Technik bekannten Blasensäule durch den Einsatz von Plasma. Hier wird das zugeführte Gas mittels eines Plasmagenerators in Plasma umgewandelt und steigt als Plasmablasen durch die Suspension. Dies ermöglicht chemische Reaktionen, die als Pyrolyse oder Plasmalyse bezeichnet werden, bei Temperaturen und Bedingungen, die in herkömmlichen Blasensäulen nicht erreichbar wären. Die die Plasmablasen umgebende Suspension erlaubt es, die hohe Energiedichte des Plasmas auf einen lokal begrenzten Bereich zu beschränken und somit sicher räumlich einzudämmen und dem in der Suspension befindlichen Abfallgranulat zuzuführen.

Hauptmerkmale des Plasma-Blasensäulenreaktors sind des Weiteren:
1. Plasmablasen: Das Gas wird in Plasma umgewandelt und steigt als Plasmablasen auf.
2. Chemische Reaktionen: Innerhalb der Plasmablasen und in der Suspension laufen Pyrolyse- oder Plasmalyse-Reaktionen ab.
3. Umwandlung von Plasmablasen: Die Plasmablasen wandeln sich in normale Gasblasen um und mischen sich mit anderen Gasen.
4. Reaktor: Der Hauptzweck ist die Durchführung von chemischen Reaktionen.
5. Plasmagenerator: Er ermöglicht die Bildung und Steuerung des Plasmas.
6. Elektrizitätszufuhrsystem: Es versorgt den Plasmagenerator mit der benötigten Energie.

Zusammengefasst kombiniert der Plasma-Blasensäulenreaktor die Vorteile der Blasensäule mit Plasmatechnologie für effiziente chemische Prozesse.

Eine Suspension bezeichnet in der Chemie und Verfahrenstechnik eine heterogene Mischung aus festen Partikeln, die in einer Flüssigkeit verteilt sind, ohne sich darin zu lösen. Die festen Partikel sind in der Flüssigkeit suspendiert, das heißt, sie schweben in der Flüssigkeit, ohne sich abzusetzen, solange die Suspension in Bewegung gehalten wird.

Im Plasma-Blasensäulenreaktor wird eine Suspension aus zerkleinerten Bio-Kunststoff-Halogenabfallmischungen und Wasser verwendet, durch die Plasmablasen aufsteigen und in der chemische Reaktionen stattfinden. Die Suspension wird durch einen Zerkleinerer erzeugt, der feste Materialien in kleinere Partikel zerlegt. Im Kontext des Plasma-Blasensäulenreaktors dient er dazu, Bio-Kunststoff-Halogenabfallmischungen für die Suspension vorzubereiten.

Ein Plasmagenerator ist ein spezialisiertes Gerät, das dazu dient, ein Gas in einen Plasma-Zustand zu versetzen. Plasma, oft als der vierte Aggregatzustand der Materie bezeichnet, besteht aus ionisierten Gaspartikeln, die sowohl positive Ionen als auch freie Elektronen enthalten. Dieser ionisierte Zustand ermöglicht eine Vielzahl von chemischen Reaktionen, die in einem normalen Gaszustand nicht möglich wären.

Im Kontext des Plasma-Blasensäulenreaktors hat der Plasmagenerator folgende Schlüsselfunktionen und Merkmale:
1. Ionisierung von Gas: Der Plasmagenerator nimmt das von unten zugeführte Gas und ionisiert es, wodurch Plasmablasen entstehen. Diese Plasmablasen steigen dann durch die Flüssigkeit in der Blasensäule auf.
2. Energieversorgung: Um das Gas in einen Plasma-Zustand zu versetzen, benötigt der Plasmagenerator eine kontinuierliche Zufuhr von elektrischer Energie. Ein spezielles Elektrizitätszufuhrsystem stellt sicher, dass dem Plasmagenerator stets die benötigte Energie zur Verfügung steht.
3. Steuerung und Regulierung: Der Plasmagenerator kann so gesteuert werden, dass er Plasma mit bestimmten Eigenschaften und in bestimmten Mengen erzeugt. Dies ermöglicht eine präzise Kontrolle über die chemischen Reaktionen, die im Plasma-Blasensäulenreaktor stattfinden.
4. Integration in den Reaktor: Der Plasmagenerator ist direkt im Plasma-Blasensäulenreaktor integriert, was eine effiziente Umwandlung des zugeführten Gases in Plasma und eine direkte Interaktion mit der umgebenden Suspension ermöglicht.
5. Förderung von Pyrolyse- und Plasmalyse-Reaktionen: Durch die Erzeugung von Plasma im Reaktor werden die Bedingungen für Pyrolyse- und Plasmalyse-Reaktionen geschaffen. Diese Reaktionen sind oft nicht nur schneller und effizienter als herkömmliche chemische Prozesse, sondern auch dadurch gekennzeichnet, dass jegliche Molekülbindung aufgelöst werden kann, da das Plasma eine hohe Energiemenge bereitstellt.

Zusammenfassend ist der Plasmagenerator ein essenzielles Element des Plasma-Blasensäulenreaktors, das die Umwandlung von Gas in Plasma ermöglicht bzw. darauf folgend den molekularen Zusammenhalt, der per Suspension zugeführten Abfallpartikel auflöst und somit die Grundlage für die spezialisierten chemischen Reaktionen innerhalb des Reaktors schafft.

Unter dem Trennungssystem wird eine Einrichtung oder ein Prozess verstanden, der dazu dient, verschiedene Komponenten eines Gemisches voneinander zu separieren. In der chemischen Verfahrenstechnik und Biotechnologie sind Trennsysteme unerlässlich, um reine Produkte aus Reaktionsgemischen zu gewinnen oder unerwünschte Nebenprodukte zu entfernen.

Im spezifischen Kontext der beschriebenen Anlage hat das Trennsystem folgende Schlüsselfunktionen und Merkmale:
1. Trennverfahren: Das Trennsystem verwendet spezialisierte Trennverfahren wie Destillation oder Umkehrosmose. Während die Destillation auf dem unterschiedlichen Siedepunkt der Komponenten basiert, um sie voneinander zu trennen, nutzt die Umkehrosmose einen selektiven Membranprozess, um Moleküle oder Ionen verschiedener Größen zu separieren.
2. Produktgewinnung: Das Hauptziel des Trennsystems ist die Trennung von Alkohol und Wasser aus dem Reaktionsgemisch. Dies ermöglicht die Gewinnung von reinem Alkohol und Wasser als separate Produkte.
3. Entstehung von Stoffströmen: Bei der Trennung des Gemisches entstehen verschiedene Stoffströme. Dazu gehören Wasser, Alkohol und unvollständig umgesetztes Alkoholsynthesegas.
4. Sauerstoffreicher Stoffstrom: Ein weiteres wichtiges Produkt des Trennsystems ist ein sauerstoffreicher Stoffstrom. Dieser kann aus Wasser, CO2 oder einer Kombination beider bestehen. Dieser Stoffstrom hat eine besondere Bedeutung, da er dem Sauerstoffabscheider zugeführt wird. Dies ermöglicht die präzise Regulierung des Sauerstoffgehalts im System, was für die nachfolgenden Prozesse von entscheidender Bedeutung ist.
5. Abtrennung von Inertgasen: Das Trennsystem verfügt auch über einen Ausgang für Gase, die nicht an der Reaktion zum Alkohol teilnehmen. Dazu gehören Spuren von Inertgasen und Stickstoff. Diese Gase werden aus dem System entfernt, um die Qualität und Effizienz des Prozesses zu gewährleisten.

Zusammenfassend ist das Trennsystem eine essenzielle Komponente der Anlage, die nicht nur die Gewinnung von reinem Alkohol und Wasser ermöglicht, sondern auch zur Regulierung des Sauerstoffgehalts im System und zur Abtrennung unerwünschter Gase beiträgt.

Ein Sauerstoffabscheider dient in diesem Verfahren dazu, Sauerstoff aus dem sauerstoffreichen Fluid (Gasgemisch oder Flüssigkeit) das aus dem Trennsystem entnommen wurde, zu entfernen. In der hier vorliegenden Erfindung kann dieser Abscheider in Form eines Elektrolyseurs oder eines CO-Elektrolyseurs realisiert werden.
1. Elektrolyseur:
   - Ein Elektrolyseur nutzt die Elektrolyse, um Wasser (H₂O) in seine Bestandteile Wasserstoff (H₂) und Sauerstoff (O₂) zu zerlegen. Dies geschieht durch Anlegen einer elektrischen Spannung an Elektroden, die in eine Elektrolytlösung eingetaucht sind.
   - An der Anode (positiv geladene Elektrode) wird Sauerstoff freigesetzt, während an der Kathode (negativ geladene Elektrode) Wasserstoff freigesetzt wird.
2. CO-Elektrolyseur:
   - Ein CO-Elektrolyseur, auch bekannt als Kohlenstoffdioxid-Elektrolyseur, ist spezialisiert auf die Umwandlung von Kohlenstoffdioxid (CO₂) in Kohlenstoffmonoxid (CO) und Sauerstoff (O₂) unter Verwendung von elektrischem Strom.
   - Ähnlich wie beim herkömmlichen Elektrolyseur wird eine elektrische Spannung an Elektroden angelegt, die in eine geeignete Elektrolytlösung eingetaucht sind.
   - An der Anode wird Sauerstoff freigesetzt, während an der Kathode Kohlenstoffmonoxid entsteht.

In beiden Fällen, ob Elektrolyseur oder CO-Elektrolyseur, wird durch den Einsatz von elektrischem Strom eine chemische Reaktion gefördert, die zur Freisetzung und somit zur Abscheidung von Sauerstoff führt.

Der Katalysator spielt eine zentrale Rolle in der Erfindung, indem er Alkoholsynthesegas effizient in Alkohol und Wasser umwandelt. Er beschleunigt die Reaktion, gewährleistet Produktqualität und arbeitet in Abstimmung mit anderen Prozesskomponenten. Nach seiner Funktion wird das Produktgemisch weiter separiert.

Das Gaseinführungssystem leitet das Gas zum Plasmagenerator, wobei es primär sauerstoffarmes Gas verwendet und die Möglichkeit bietet, Rückführungsgase wie z.B. nicht umgesetztes Alkoholsynthesegas zu integrieren. Es stellt sicher, dass die Bedingungen für die Plasmabildung optimal sind und bietet gleichzeitig Sicherheit und Anpassungsfähigkeit an verschiedene Abfallmaterialien.

Das Rückführsystem in der beschriebenen Erfindung ist ein einfacher, aber essenzieller Mechanismus, der für die effiziente Rückführung eines sauerstoffreichen Stoffstroms aus dem Trennungssystem in den Sauerstoffabscheider zuständig ist. Das System leitet den aus dem Trennungssystem entnommenen sauerstoffreichen Stoffstrom, der Wasser, CO2 oder eine Mischung aus beidem enthalten kann, gezielt in den Sauerstoffabscheider.

Das Wasser-Recycling-System spielt eine zentrale Rolle bei der effizienten Nutzung und Wiederverwendung von Wasser innerhalb der beschriebenen Erfindung. Nachdem das Wasser in der Asche-/Schlacke-Trennungseinheit von der Asche oder Schlacke getrennt wurde, wird es über das Wasser-Recycling-System zurück in das Suspensionssystem geleitet. Hier wird das recycelte Wasser mit der granulären Bio-Kunststoff-Halogenabfallmischung und mit extern zugeführtem Wasser kombiniert, um eine Suspension zu bilden.

Die Asche-/Schlacke-Trennungseinheit ist für die Separation von Wasser und Asche oder Schlacke verantwortlich. Die Einheit nimmt eine Asche-/Schlacke-Suspension aus dem Plasma-Blasensäulenreaktor auf und trennt das Wasser von den festen Bestandteilen. Dies kann beispielsweise durch Filtration, Zentrifugation, Sedimentation oder Dekantieren erfolgen. Das resultierende Wasser wird dann über das Wasser-Recycling-System zurückgeführt, während die getrennte Asche oder Schlacke für weitere Verarbeitungsschritte oder Entsorgung bereitsteht.

Unter Stoffrückführungen versteht man ein System oder eine Methode, die darauf abzielt, bestimmte Materialien oder Substanzen, die während eines Prozesses nicht vollständig umgesetzt oder genutzt wurden, wieder in den ursprünglichen oder einen nachfolgenden Prozessschritt zurückzuführen. Dies dient dazu, die Effizienz des Gesamtprozesses zu erhöhen, Ressourcen zu schonen und Abfall oder Verluste zu minimieren.

Im Kontext der vorliegenden Erfindung bezieht sich die Stoffrückführung speziell auf das nicht umgesetzte Alkoholsynthesegas. Dieses Gas wird im Trennungssystem abgeschieden und anstatt es als Abfall zu behandeln oder freizusetzen, wird es über das Stoffrückführungssystem wieder in den Prozess zurückgeführt. Dadurch wird sichergestellt, dass das Alkoholsynthesegas maximal genutzt wird und der Prozess insgesamt effizienter und ressourcenschonender abläuft.

Die Verwendung von Stoffrückführungen ist ein Zeichen für ein nachhaltiges und umweltbewusstes Prozessdesign, da sie dazu beiträgt, den Verbrauch von Rohstoffen zu reduzieren und die Umweltauswirkungen zu minimieren.

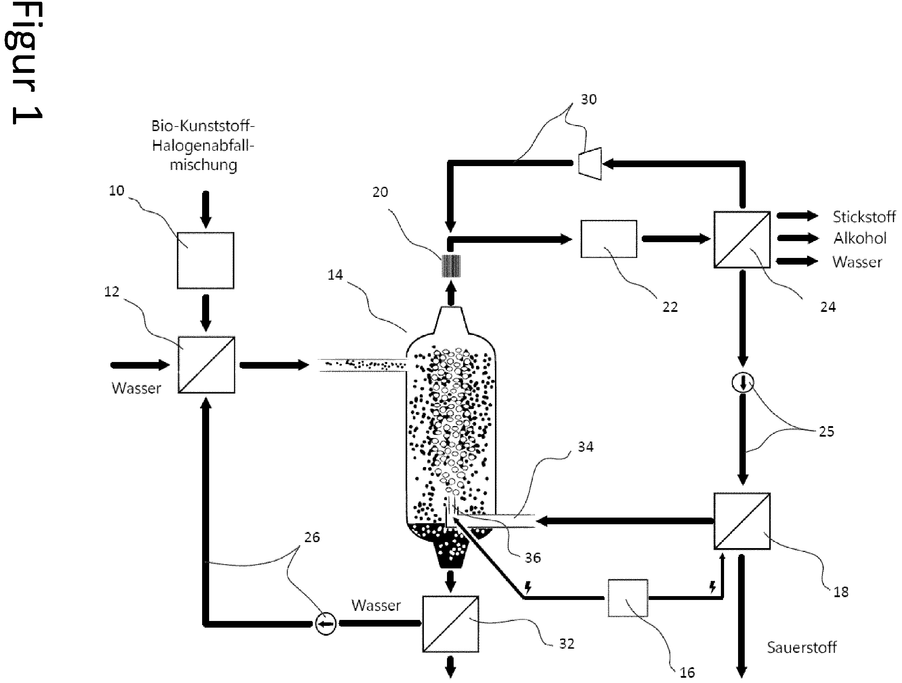

Nachfolgend wird eine bevorzugte Ausführungsform der vorliegenden Erfindung anhand der Figur näher beschrieben.

Die Figur zeigt eine schematische Ansicht einer Anlage zur Herstellung von Alkohol aus heterogenen Bio-Kunststoff-Halogenabfallmischungen gemäß einer bevorzugten Ausführungsform der vorliegenden Erfindung.

Die Anlage weist einen Zerkleinerer (10) für Bio-Kunststoff-Halogenabfallmischungen, ein Suspensionssystem (12) zur Bildung einer Suspension aus zerkleinertem Bio-Kunststoff-Halogenabfall-Granulat und Wasser, einen Plasma-Blasensäulenreaktor (14), einen darin integrierten Plasmagenerator (36), ein Elektrizitätszufuhrsystem (16) zur Energieversorgung des Plasmagenerators (36), einen Sauerstoffabscheider (18) zur Kontrolle des C:H:O-Verhältnisses der Mischung, einen Halogenwasserstoff-Neutralisator (20) zur Reduzierung von Halogenwasserstoff-Verunreinigungen, einen Katalysator (22) zur Umwandlung des Alkoholsynthesegases in ein Alkohol-Wasser-Gemisch, ein Trennungssystem (24) zur Trennung von Alkohol, Wasser und Alkoholsynthesegas, ein Rückführsystem (25) zur Rückführung eines sauerstoffreichen Fluids, ein Wasser-Recycling-System (26) zum Wiederverwenden des Wassers aus dem Prozess, eine Asche-/Schlacke-Trennungseinheit (32) zur Trennung von Asche oder Schlacke, und ein Gaseinführungssystem (34) für sauerstoffarmes Gas aus dem Sauerstoffabscheider (18) in den Plasma-Blasensäulenreaktor (14) auf.

Dem Zerkleinerer (10), bei dem es sich bspw. um eine Hammermühle, ein Schneidmühlen-System oder eine Kugelmühle handeln kann, wird entweder manuell oder automatisch eine Bio-Kunststoff-Halogenabfallmischung zugeführt. Letztere wird im Zerkleinerer auf Partikel einer Größe von 1 mm bis 30 mm zerkleinert. Die zerkleinerten Partikel werden dann im Suspensionssystem, bei dem es sich bspw. um ein Paddelrührwerk, ein Trommelmischsystem oder ein Schneckenmischsystem handeln kann, mit Wasser versetzt, um eine Bio-Kunststoff-Halogenabfallmischungspartikel-Wassersuspension herzustellen. Das Verhältnis von Feststoffen zu Wasser in der Suspension beträgt dabei bevorzugt zwischen 1:10 und 1:50.

Das für die Suspension benötigte Wasser stammt sowohl aus externen Quellen (vgl. den Pfeil links) als auch aus dem Wasser-Recycling-System (26), das Wasser aus der Asche-/Schlacke-Trennungseinheit (32) zurückführt. Die Menge des zugeführten Wassers orientiert sich an der Beschaffenheit der Bio-Kunststoff-Halogenabfallmischung. Als Richtwert dient das C:H-Verhältnis eines trockenen Ausgangssubstrats, wobei das angestrebte Verhältnis im Alkoholsynthesegas (bei Methanol) 1:4 ist. Aufgrund variierender Zusammensetzungen des Abfalls wird von einem durchschnittlichen C:H-Verhältnis von 1:2 ausgegangen. Der Wassergehalt des Abfalls wird geschätzt und typischerweise als 1:1-Mischung aus Trockenmasse und Wasser betrachtet. Die im Betrieb benötigte Menge an externem Wasser wird durch eine rückwärtige Bilanzierung ermittelt, die auf dem produzierten Alkohol und der Zusammensetzung des Alkoholsynthesegases basiert. Die Stoffströme, die in das Trennungssystem (24) ein- und ausfließen, werden kontinuierlich mit geeigneten Messgeräten überwacht, wie beispielsweise solchen, die auf Infrarot-Spektroskopie (IR), Tunable Diode Laser Absorption Spectroscopy (TDLAS) oder Fourier-Transform-Infrarot-Spektroskopie (FTIR) basieren. Je nach Bilanz wird das benötigte Wasser dem Suspensionssystem (12) zugeführt. Sollte der Abfall einen hohen Wassergehalt haben, kann es erforderlich sein, Wasser aus dem System zu entfernen. In solch einem Fall wird kein externes Wasser hinzugefügt, sondern Wasser aus dem Trennungsprozess an die Umgebung abgeführt. Die gebildete Suspension aus Bio-Kunststoff-Halogenabfall und Wasser wird dem Plasma-Blasensäulenreaktor (PBSR) (14) zur weiteren Umsetzung zugeführt. Dem Plasma-Blasensäulenreaktor (14) wird des Weiteren das sauerstoffarme Gas über das Gaseinführungssystem (34) zugegeben. Im PBSR (14) entsteht eine Dreiphasenströmung, bestehend aus der Gasphase (Plasma und Gas), der Feststoffphase (Abfallpartikel) und der Flüssigphase (Wasser der Suspension). Durch Dichteunterschiede dieser Phasen entstehen im PBSR (14) Zirkulationsströmungen, die für eine stetige Durchmischung der Substrate sorgen. Innerhalb der Gasblasen bilden sich ebenfalls Zirkulationsströmungen, wodurch Partikel an die Blasenoberfläche transportiert und dort mit der umgebenden Wasserphase koalesziert werden. Sowohl flüssige als auch feste Partikel in der Gasphase werden von der Flüssigphase aufgenommen, was zu einem Reinigungseffekt des Gases in der Blase führt. Am PBSR-Ausgang liegt ein Gasgemisch (Alkoholsynthesegas) vor, das nur aus den bei ca. 150-200 °C flüchtigen Gasen besteht.

Wie der Name PBSR bereits suggeriert, dominiert im Reaktor nicht ausschließlich ein fest-flüssiggas Regime. Insbesondere im Bereich des Plasmagenerators (36) entsteht ein Plasma-Gas-Flüssig-Feststoff-System. Das über das Gaseinführungssystem (34) zugeführte, sauerstoffarme Gas wird mittels Plasmagenerator, genauer gesagt mittels eines Schweißbrenners nach dem Arcatom-Schweißverfahren (Langmuir-Fackel), durch elektrischen Strom in Plasma umgewandelt.

Die Plasmablasen steigen aufgrund von Dichteunterschieden im PBSR auf und sorgen für eine konstante Durchmischung. Die hohe Plasmaenergie wird an die Umgebung übertragen, wodurch der Bio-Kunststoff-Halogenabfall in der Suspension getrocknet, verdampft und schließlich atomar dissoziiert wird. Es entsteht eine dreiphasige Strömung aus Gas, Flüssigkeit und festen Partikeln. Durch Rückvermischungen, Zirkulationsströmungen und resultierende Fliehkräfte erfolgt eine Trennung in leichte und schwere sowie flüchtige und weniger flüchtige Verbindungen. Die Reaktion der Abfallpartikel mit dem Plasma erzeugt Gas und Asche- bzw. Schlackepartikel. Das hohe Wasser-Feststoff-Verhältnis in der Suspension gewährleistet, dass die Plasma/Gas-Phase volumenmäßig kleiner bleibt als das umgebende Wasser, wodurch die Suspension einerseits fließfähig bleibt und andererseits die Plasmazone sicher umschlossen bleibt. Aufgrund des hohen Wassergehaltes und aufgrund des Betriebsdruckes innerhalb der PBSR wird die Temperatur der PBRS auf einem Temperaturniveau um 183 °C gehalten (Siedepunkt des Wasser bei 10 bar Behälterdruck). Die schwereren Asche-/Schlackepartikel sammeln sich am PBSR-Boden und können dort entnommen werden. Diese Asche-/Schlacke-Wasser-Suspension wird zur Asche-/Schlacke-Trennungseinheit geleitet und dort in Wasser und Asche bzw. Schlacke separiert. Die Trennung erfolgt durch eine Kombination aus Filtration (zur Partikelabtrennung) und Umkehrosmose (zur Salzabtrennung aus dem Wasser).

Das abgetrennte Wasser wird dem Suspensionssystem zurückgeführt und durchläuft den Prozess erneut. An der Spitze verlässt die Gasphase, also das Alkoholsynthesegas, den PBSR. Da das Alkoholsynthesegas am PBSR-Austritt durch Halogenwasserstoffe kontaminiert sein kann, wird es dem Halogenwasserstoff-Neutralisator (20) zugeleitet. In diesem Neutralisator reagieren die Halogenwasserstoffe mit dem Neutralisator-Material, beispielsweise Calciumcarbonat oder Natriumcarbonat. Diese Stoffe reagieren aufgrund ihrer chemischen Eigenschaften mit den Halogenwasserstoffen und werden in harmloses Salz, Wasser und CO2 umgewandelt. Der Halogenwasserstoff-Neutralisator (20) ist somit ein Verbrauchsmaterial und muss regelmäßig ausgetauscht werden. Nach der Behandlung im Halogenwasserstoff-Neutralisator (20) resultiert ein Alkoholsynthesegas, das von gefährlichen und stark korrosiven Bestandteilen befreit ist. Dieses gereinigte Gas wird im Katalysator, der beispielhafterweise aus einer Schüttung von Eisenkugeln besteht, in Alkohol und Wasser umgewandelt. Da die Umwandlung des Alkoholsynthesegases in Wasser und Alkohol exotherm verläuft, muss der Katalysator gekühlt werden. Es gibt bereits etablierte Kühlmethoden, aber im Kontext dieser Erfindung könnte auch eine prozessinterne Energieverwertung zum Einsatz gebracht werden.

Das aus dem Katalysator extrahierte Stoffgemisch besteht aus Wasser, Alkohol und den nicht vollständig umgesetzten Edukten, also den Resten des Alkoholsynthesegases. Um Wasser und Alkohol von den Synthesegasresten zu separieren, wird das Gemisch vorzugsweise auf 40 °C oder darunter abgekühlt. Für diese Gas-Flüssig-Trennung eignet sich ein Abscheider oder ein Separator. Doch diese Trennung repräsentiert nur einen Aspekt der Aufgaben des Trennungssystems (24). Das System verfügt über weitere Komponenten, die gezielt zur Abtrennung von Stickstoff und anderen Inertgasen konzipiert sind. Die Stickstoffabtrennung kann mittels Druckwechseladsorption (PSA) oder geeigneten Membrantrennverfahren erfolgen. Auch Destillationsverfahren oder kombinierte Techniken aus Adsorption/Desorption und Destillation sind anwendbar. Es ist besonders zu betonen, dass das Trennungssystem auch zur Herstellung von technisch reinem Alkohol (mit einer Reinheit von ca. 98 Vol%) und technisch reinem Wasser (mit einer Reinheit von ca. 99,9 Massen%) dient. Daher stellt eine kombinierte Destillationsanlage hier die bevorzugte Methode dar. Am Trennungssystem werden sowohl die ein- als auch die ausströmenden Massen und ihre Zusammensetzung genau gemessen. Zusammen mit dem gemessenen Massenstrom der Bio-Kunststoff-Halogenabfallmischung ermöglicht eine atomare Bilanzierung die genaue Bestimmung der benötigten Wassermenge, die entweder am Suspensionssystem (12) von extern hinzugefügt oder am Trennungssystem entfernt werden muss (Einstellung des C:H- Verhältnisses). Diese Bilanzierung gibt auch Aufschluss über die Menge des im Sauerstoffabscheider (18) zu entfernenden Sauerstoffs (Einstellung des C:H:O- Verhältnisses). Die Bilanzierung wird von einem Rechner vorgenommen und dann für die Regelung der Anlage bzw. der Produktqualität verwendet.

Als letzter integraler Prozessschritt ist der Sauerstoffabscheider (18) zu sehen, der z.B. in Form eines Elektrolysesystems ausgeführt werden kann. Gemäß der von einem Rechner (bzw. Regler) vorgegebenen Menge des abzutrennenden Sauerstoffes wird dem Sauerstoffabscheider (18) über das Rückführsystem (25) ein sauerstoffreicher Stoffstrom zugeführt. Vorzugsweise handelt es sich bei diesem als "sauerstoffreichen Stoffstrom" bezeichneten Strom um reines Wasser.

### Liste der Bezugszeichen:

10 - Zerkleinerer
12 - Suspensionssystem
14 - Plasma-Blasensäulenreaktor
16 - Elektrizitätszufuhrsystem
18 - Sauerstoffabscheider
20 - Halogenwasserstoff-Neutralisator
22 - Katalysator
24 - Trennungssystem
25 - Rückführsystem
26 - Wasser-Recycling-System
30 - Stoffrückführungen
32 - Asche-/Schlacke-Trennungseinheit
34 - Gaseinführungssystem
36 - Plasmagenerator

## Patentansprüche

1. Anlage zur Herstellung von Alkohol aus heterogenen Bio-Kunststoff-Halogenabfallmischungen, wobei die Bio-Kunststoff-Halogenabfallmischungen sowohl biologische Abfälle als auch Kunststoffabfälle und halogenhaltige Verbindungen enthalten, wobei die Anlage umfasst:
i. einen Zerkleinerer (10) für Bio-Kunststoff-Halogenabfallmischungen,
ii. ein Suspensionssystem (12) zur Bildung einer Suspension aus zerkleinertem Bio-Kunststoff-Halogenabfall-Granulat und Wasser,
iii. einen Plasma-Blasensäulenreaktor (14) mit einem Plasmagenerator (36),
iv. einen Sauerstoffabscheider (18), der dazu geeignet ist, Sauerstoff aus einem sauerstoffreichen Fluid zu entfernen,
v. einen Halogenwasserstoff-Neutralisator (20),
vi. einen Katalysator (22) zur Umwandlung des aus dem Halogenwasserstoff-Neutralisator (20) austretenden Alkoholsynthesegases in ein Gemisch mit Alkohol und Wasser,
vii. ein Gaseinführungssystem (34) zur Einführung von Gas in die im Plasma-Blasensäulenreaktor (14) befindliche Suspension aus zerkleinertem Bio-Kunststoff-Halogenabfall-Granulat und Wasser,
viii. ein Trennungssystem (24), welches das aus dem Katalysator (22) entnommene Stoffgemisch in separate Stoffströme, einschließlich aber nicht beschränkt auf Alkohol, Wasser, Inertgase, Stickstoff, nicht umgesetztes Alkoholsynthesegas sowie einen sauerstoffreichen Stoffstrom, der H₂O und/oder CO₂ aufweist, aufteilt, und
ix. ein Rückführsystem (25) zur Überführung eines sauerstoffreichen Stoffstroms aus dem Trennungssystem (24) in den Sauerstoffabscheider (18);
wobei die Anlage dazu geeignet ist, sauerstoffarmes Gas aus dem Sauerstoffabscheider (18) in den Plasma-Blasensäulenreaktor (14) einzuführen, um eine 3-Phasen-Dispersion zu bilden.

2. Anlage nach Anspruch 1, wobei der Sauerstoffabscheider (18) ein Elektrolyseur oder ein CO-Elektrolyseur ist.

3. Anlage nach einem der vorhergehenden Ansprüche, weiterhin umfassend: Stoffrückführungen (30) zur Rückführung von nicht umgesetztem Alkoholsynthesegas aus dem Trennungssystem (24) entweder an eine Stelle vor dem Katalysator (22) oder über das Gaseinführungssystem (34) in den Plasma-Blasensäulenreaktor (14).

4. Anlage nach einem der vorhergehenden Ansprüche, weiterhin umfassend: eine Asche-/Schlacke-Trennungseinheit (32) und ein Wasser-Recycling-System (26) zur Rückführung von Wasser aus der Asche-/Schlacke-Trennungseinheit (32) in das Suspensionssystem (12).

5. Anlage nach einem der vorhergehenden Ansprüche, weiterhin umfassend: eine Zuleitung, die zur Zufuhr von externem Wasser in das Suspensionssystem (12) dient und/oder eine Abfuhr von Wasser aus dem Trennungssystem (24) an die Umgebung ermöglicht.

6. Verfahren zur Alkoholproduktion aus heterogenen Bio-Kunststoff-Halogenabfallmischungen, wobei die Bio-Kunststoff-Halogenabfallmischungen sowohl biologische Abfälle als auch Kunststoffabfälle und halogenhaltige Verbindungen enthalten, mit den folgenden Schritten:
i. Aufbereiten der Bio-Kunststoff-Halogenabfallmischungen durch Zerkleinerung mittels eines Zerkleinerers (10),
ii. Kombinieren des zerkleinerten Materials mit Wasser mittels eines Suspensionssystems (12) zur Erzeugung einer Suspension,
iii. Einbringen der Suspension in einen Plasma-Blasensäulenreaktor (14) und Erzeugen eines Plasmas im Plasma-Blasensäulenreaktor (14) zur Behandlung der Suspension,
iv. Neutralisieren von Halogenwasserstoff-Verunreinigungen in dem aus der Plasma-Blasensäule entnommenen, resultierenden Synthesegas mittels eines Halogenwasserstoff-Neutralisators (20),
v. Katalysieren des Synthesegases zur Bildung eines Alkohol-Wasser-Gemisches mittels eines Katalysators (22),
vi. Abtrennen von Stickstoff, Alkohol und Wasser aus dem katalysierten Synthesegas in einem Trennungssystem (24), wobei in dem Trennungssystems (24) das aus dem Katalysator (22) entnommenen Stoffgemisch in separate Stoffströme, einschließlich aber nicht beschränkt auf Alkohol, Wasser, Inertgase, Stickstoff, nicht umgesetztes Alkoholsynthesegas sowie einen sauerstoffreichen Stoffstrom, der H₂O und/oder CO₂ aufweist, getrennt wird, und
vii. Abscheiden von Sauerstoff aus dem dem Trennungssystem (24) entnommenen Stoffstrom in einem Sauerstoffabscheider (18), der dazu geeignet ist, Sauerstoff aus einem sauerstoffreichen Fluid zu entfernen, und
viii. Einführen von sauerstoffarmem Gas aus dem Sauerstoffabscheider (18) in den Plasma-Blasensäulenreaktor (14), um eine 3-Phasen-Dispersion zu bilden.

7. Verfahren nach Anspruch 6, wobei der Sauerstoffabscheider (18) ein Elektrolyseur oder ein CO-Elektrolyseur ist.

8. Verfahren nach Anspruch 6 oder 7, weiterhin umfassend: Rückführen von nicht umgesetztem Alkoholsynthesegas aus dem Trennungssystem (24) entweder an eine Stelle vor dem Katalysator (22) oder über ein Gaseinführungssystem (34) in den Plasma-Blasensäulenreaktor (14) mittels Stoffrückführungen (30).

9. Verfahren nach einem der Ansprüche 6-8, wobei dem Plasma-Blasensäulenreaktor (14) ein Stoffgemisch entnommen und einer Asche-/Schlacke-Trennungseinheit (32) zugeführt wird, wobei bevorzugt Wasser aus der Asche-/Schlacke-Trennungseinheit (32) in das Suspensionssystem (12) mittels eines Wasser-Recycling-Systems (26) rückgeführt wird.

10. Verfahren nach einem der Ansprüche 6-9, wobei ferner ein sauerstoffreiches Fluid aus dem Trennungssystem (24) in den Sauerstoffabscheider (18) rückgeführt wird.

11. Verfahren nach einem der Ansprüche 6-10, wobei ferner externes Wasser in das Suspensionssystem (12) zugeführt wird und/oder Wasser aus dem Trennungssystem (24) an die Umgebung abgeführt wird.

## Claims

1. System for producing alcohol from heterogeneous bio-plastic-halogen waste mixtures, wherein the bio-plastic-halogen waste mixtures contain both biological waste and plastic waste and halogenated compounds, the system comprising:
i. a shredder (10) for bio-plastic-halogen waste mixtures,
ii. a suspension system (12) for forming a suspension of shredded bio-plastic-halogen waste granules and water,
iii. a plasma bubble column reactor (14) with a plasma generator (36),
iv. an oxygen separator (18) suitable for removing oxygen from an oxygen-rich fluid,
v. a hydrogen halide neutralizer (20),
vi. a catalyst (22) for converting the alcohol synthesis gas exiting the hydrogen halide neutralizer (20) into a mixture of alcohol and water,
vii. a gas feed system (34) for introducing gas into the suspension of shredded bio-plastic-halogen waste granules and water in the plasma bubble column reactor (14),
viii. a separation system (24) that divides the mixture of substances taken from the catalyst (22) into separate material streams, including but not limited to alcohol, water, inert gases, nitrogen, unconverted alcohol synthesis gas, and an oxygen-rich stream comprising H₂O and/or CO₂, and
ix. a return system (25) for directing an oxygen-rich material stream from the separation system (24) to the oxygen separator (18),
wherein the system is suitable for introducing oxygen-depleted gas from the oxygen separator (18) into the plasma bubble column reactor (14) to form a three-phase dispersion.

2. The system of claim 1, wherein the oxygen separator (18) is an electrolyzer or a CO electrolyzer.

3. The system of any one of the preceding claims, further comprising:
recirculation units (30) for returning unconverted alcohol synthesis gas from the separation system (24) either to a point before the catalyst (22) or into the plasma bubble column reactor (14) via the gas feed system (34).

4. The system of any one of the preceding claims, further comprising:
an ash/sludge separation unit (32) and a water recycling system (26) for returning water from the ash/sludge separation unit (32) to the suspension system (12).

5. The system of any one of the preceding claims, further comprising:
a supply line for introducing external water into the suspension system (12) and/or for discharging water from the separation system (24) into the environment.

6. Method for producing alcohol from heterogeneous bio-plastic-halogen waste mixtures, wherein the bio-plastic-halogen waste mixtures contain both biological waste and plastic waste and halogenated compounds, comprising the steps of:
i. preparing the bio-plastic-halogen waste mixtures by shredding them using a shredder (10),
ii. combining the shredded material with water using a suspension system (12) to form a suspension,
iii. introducing the suspension into a plasma bubble column reactor (14) and generating plasma in the plasma bubble column reactor (14) to treat the suspension,
iv. neutralizing hydrogen halide impurities in the resulting synthesis gas taken from the plasma bubble column using a hydrogen halide neutralizer (20),
v. catalyzing the synthesis gas to form an alcohol-water mixture using a catalyst (22),
vi. separating nitrogen, alcohol, and water from the catalyzed synthesis gas in a separation system (24), wherein the mixture of substances taken from the catalyst (22) is separated into individual streams, including but not limited to alcohol, water, inert gases, nitrogen, unconverted alcohol synthesis gas, and an oxygen-rich stream comprising H₂O and/or CO₂, and
vii. removing oxygen from the material stream taken from the separation system (24) using an oxygen separator (18), suitable for removing oxygen from an oxygen-rich fluid, and
viii.introducing oxygen-depleted gas from the oxygen separator (18) into the plasma bubble column reactor (14) to form a three-phase dispersion.

7. The method of claim 6, wherein the oxygen separator (18) is an electrolyzer or a CO electrolyzer.

8. The method of claim 6 or 7, further comprising:
returning unconverted alcohol synthesis gas from the separation system (24) either to a point before the catalyst (22) or, via a gas feed system (34), into the plasma bubble column reactor (14) using recirculation units (30).

9. The method of any one of claims 6 to 8, wherein a mixture of substances is extracted from the plasma bubble column reactor (14) and directed to an ash/sludge separation unit (32), and preferably water from the ash/sludge separation unit (32) is returned to the suspension system (12) via a water recycling system (26).

10. The method of any one of claims 6 to 9, further comprising:
returning an oxygen-rich fluid from the separation system (24) to the oxygen separator (18).

11. The method of any one of claims 6 to 10, further comprising:
introducing external water into the suspension system (12) and/or discharging water from the separation system (24) into the environment.

## Revendications

1. **Installation pour la production d'alcool à partir de mélanges de déchets hétérogènes bio-plastiques-halogènes,** les mélanges **de déchets hétérogènes bio-plastiques-halogènes** contenant à la fois des déchets biologiques et des déchets plastiques et des composés halogénés, ladite installation comprenant :
i. un broyeur (10) pour les mélanges de déchets bio-plastiques-halogènes,
ii. un système de suspension (12) pour former une suspension de granulés broyés de déchets bio-plastiques-halogènes et d'eau,
iii. un réacteur à colonne à bulles plasma (14) avec un générateur de plasma intégré (36),
iv. un séparateur d'oxygène (18) apte à éliminer l'oxygène d'un fluide riche en oxygène,
v. un neutraliseur d'hydrogénures halogénés (20),
vi. un catalyseur (22) pour convertir le gaz de synthèse alcoolique sortant du neutraliseur d'hydrogénures halogénés (20) en un mélange d'alcool et d'eau,
vii. un système d'alimentation en gaz (34) pour introduire du gaz dans la suspension de granulés broyés de déchets bio-plastiques-halogènes et d'eau dans le réacteur à colonne à bulles plasma (14),
viii.un système de séparation (24) qui divise le mélange de substances extrait du catalyseur (22) en différents flux de substances, incluant, sans s'y limiter, de l'alcool, de l'eau, des gaz inertes, de l'azote, du gaz de synthèse alcoolique non converti ainsi qu'un flux riche en oxygène contenant H₂O et/ou CO₂, et
ix. un système de recirculation (25) pour diriger un flux de substances riche en oxygène du système de séparation (24) vers le séparateur d'oxygène (18), ladite installation étant apte à introduire du gaz appauvri en oxygène depuis le séparateur d'oxygène (18) dans le réacteur à colonne à bulles plasma (14) pour former une dispersion triphasique.

2. **Installation selon la revendication 1,** dans laquelle le séparateur d'oxygène (18) est un électrolyseur ou un électrolyseur de CO.

3. **Installation selon l'une des revendications précédentes,** comprenant en outre :
des unités de recirculation (30) pour renvoyer le gaz de synthèse alcoolique non converti depuis le système de séparation (24), soit en amont du catalyseur (22), soit via le système d'alimentation en gaz (34) dans le réacteur à colonne à bulles plasma (14).

4. **Installation selon l'une des revendications précédentes,** comprenant en outre :
une unité de séparation des cendres/des scories (32) et un système de recyclage de l'eau (26) pour retourner l'eau de l'unité de séparation des cendres/des scories (32) au système de suspension (12).

5. **Installation selon l'une des revendications précédentes,** comprenant en outre :
une conduite d'alimentation pour introduire de l'eau externe dans le système de suspension (12) et/ou pour rejeter de l'eau du système de séparation (24) vers l'environnement.

6. **Procédé de production d'alcool à partir de mélanges de déchets hétérogènes bio-plastiques-halogènes,** lesdits mélanges contenant à la fois des déchets biologiques et des déchets plastiques et des composés halogénés, comprenant les étapes suivantes :
i. préparation des mélanges de déchets bio-plastiques-halogènes par broyage au moyen d'un broyeur (10),
ii. combinaison du matériau broyé avec de l'eau au moyen d'un système de suspension (12) pour former une suspension,
iii. introduction de la suspension dans un réacteur à colonne à bulles plasma (14) et génération de plasma dans le réacteur à colonne à bulles plasma (14) pour traiter la suspension,
iv. neutralisation des impuretés d'hydrogénures halogénés dans le gaz de synthèse résultant extrait de la colonne à bulles plasma par un neutraliseur d'hydrogénures halogénés (20),
v. catalyse du gaz de synthèse pour former un mélange alcool-eau au moyen d'un catalyseur (22),
vi. séparation de l'azote, de l'alcool et de l'eau à partir du gaz de synthèse catalysé dans un système de séparation (24), dans lequel le mélange de substances extrait du catalyseur (22) est séparé en différents flux, incluant sans s'y limiter alcool, eau, gaz inertes, azote, gaz de synthèse non converti, et un flux riche en oxygène contenant H₂O et/ou CO₂, et
vii. élimination de l'oxygène du flux de substances extrait du système de séparation (24) au moyen d'un séparateur d'oxygène (18) apte à éliminer l'oxygène d'un fluide riche en oxygène, et
viii.introduction de gaz appauvri en oxygène depuis le séparateur d'oxygène (18) dans le réacteur à colonne à bulles plasma (14) pour former une dispersion triphasique.

7. **Procédé selon la revendication 6,** dans lequel le séparateur d'oxygène (18) est un électrolyseur ou un électrolyseur de CO.

8. **Procédé selon la revendication 6 ou 7,** comprenant en outre :
le renvoi du gaz de synthèse alcoolique non converti du système de séparation (24), soit en amont du catalyseur (22), soit via un système d'alimentation en gaz (34), dans le réacteur à colonne à bulles plasma (14), au moyen des unités de recirculation (30).

9. **Procédé selon l'une des revendications 6 à 8,** dans lequel un mélange de substances est extrait du réacteur à colonne à bulles plasma (14) et dirigé vers une unité de séparation des cendres/des scories (32), et dans lequel de préférence l'eau provenant de la unité de séparation des cendres/des scories (32) est renvoyée vers le système de suspension (12) au moyen d'un système de recyclage de l'eau (26).

10. **Procédé selon l'une des revendications 6 à 9,** comprenant en outre :
le renvoi d'un fluide riche en oxygène du système de séparation (24) vers le séparateur d'oxygène (18).

11. **Procédé selon l'une des revendications 6 à 10,** comprenant en outre :
l'introduction d'eau externe dans le système de suspension (12) et/ou l'évacuation de l'eau du système de séparation (24) vers l'environnement.
